(19) Europäisches Patentamt
European Patent Office
Office européen des brevets

(11) Veröffentlichungsnummer: **0 659 731 A1**

# (12) EUROPÄISCHE PATENTANMELDUNG

(21) Anmeldenummer: **94119607.3**

(22) Anmeldetag: **12.12.94**

(51) Int. Cl.⁶: **C07C 68/00**, C07C 69/96

(30) Priorität: **23.12.93 DE 4344159**

(43) Veröffentlichungstag der Anmeldung:
**28.06.95 Patentblatt 95/26**

(84) Benannte Vertragsstaaten:
**BE CH DE ES FR GB IT LI NL**

(71) Anmelder: **BAYER AG**

**D-51368 Leverkusen (DE)**

(72) Erfinder: **Kricsfalussy, Zoltan, Dr.**
**Franz-Marc-Strasse 32**
**D-51375 Leverkusen (DE)**
Erfinder: **Steude, Heinrich, Dipl.-Ing.**
**Am Telegraf 24**
**D-51375 Leverkusen (DE)**
Erfinder: **Waldmann, Helmut, Dr.**
**Henry-T.-V.-Böttinger Strasse 15**
**D-51373 Leverkusen (DE)**
Erfinder: **Hallenberger, Kaspar, Dipl.-Ing.**
**Vincent-von-Gogh-Strasse 44**
**D-51375 Leverkusen (DE)**
Erfinder: **Wagner, Wolfram, Dr.**
**Zeisigstrasse 9**
**D-41540 Dormagen (DE)**
Erfinder: **Traenckner, Hans-Joachim, Dr.**
**An der Steinrütsch 30**
**D-51375 Leverkusen (DE)**

## (54) Verfahren zur Herstellung von Dimethylcarbonat.

(57) Bei der Herstellung von Dimethylcarbonat durch Umsetzung von Methanol, Kohlenmonoxid und Sauerstoff in Gegenwart von Cu-Verbindungen wird erfindungsgemäß in Destillationseinheiten gearbeitet und das Reaktionswasser mindestens teilweise aus dem Abtriebsbereich oder der Sumpfphase ausgeschleust.

EP 0 659 731 A1

Die Erfindung betrifft ein verbessertes Verfahren zur Herstellung von Dimethylcarbonat (DMC) durch Oxycarbonylierung von Methanol in Gegenwart von Cu-Salzen.

Dimethylcarbonat ist ein Zwischenprodukt mit geringer Toxizität und kann giftige Zwischenprodukte, wie Phosgen oder Dimethylsulfat, bei vielen Reaktionen ersetzen. Es ist ferner nicht korrosiv. Bei seiner Verwendung entstehen keine umweltschädlichen Nebenprodukte.

Beispiele solcher Reaktionen von Dimethylcarbonat sind die Herstellung von Urethanen aus aliphatischen oder aromatischen Aminen, die wiederum zu den entsprechenden Isocyanaten gespalten werden können. Dimethylcarbonat kann beispielsweise auch Dimethylsulfat bei der Quaternisierung von Aminen oder bei der Methylierung von Phenol oder von Naphtholen ersetzen. Dimethylcarbonat kann ferner dem Fahrbenzin als Oktanzahl-Verbesserer zugesetzt werden, z.B. anstelle von Bleiverbindungen. Angesichts dieser Bedeutung von Dimethylcarbonat ist die Suche nach einem technisch einfachen und umweltverträglichen Produktionsverfahren, das für große Kapazitäten ohne wesentliche Nebenproduktbildung oder gekoppelte Stoffkreisläufe geeignet ist, angezeigt.

Zur Herstellung von Dimethylcarbonat gibt es verschiedene, im kleinen Maßstab bereits auch technisch erprobte Herstellungsverfahren. Die Herstellungswege, die auf der katalytischen Umsetzung von Methanol mit Kohlenmonoxid und Sauerstoff gemäß nachstehender Gleichung beruhen, sind von verschiedenen Arbeitsgruppen intensiv bearbeitet worden:

$$2\ CH_3OH + CO + 1/2\ O_2 \xrightarrow[\text{salze}]{\text{Kupfer-}} CH_3O\text{-}CO\text{-}OCH_3 + H_2O$$

So hat man die als Katalysatoren wirkenden Kupferverbindungen in Form verschiedener Kupfersalze eingesetzt. Bei der Verwendung von Kupfer-II-chlorid als Katalysator gemäß JP-45/11129 (1970) erreicht man unbefriedigende Selektivitäten. Störend ist vor allem die Bildung größerer Mengen an Methylchlorid, das wegen seiner großen Flüchtigkeit dazu neigt, sich ubiquitär in der ganzen Produktionsanlage zu verteilen und praktisch in der gesamten Anlage zu Korrosionen führen kann.

Bessere Selektivitäten erhält man bei der Verwendung von organischen Komplexbildnern (DE-A 21 10 194), aber dort hat man das Problem der Abtrennung der Katalysatorsalze, die im Reaktionsgemisch teils gelöst sind, zum größeren Teil aber als Suspension vorliegen.

Ganz besonders problematisch ist die Durchführung dieser Reaktion gemäß DE-A 27 43 690, da die Katalysatorsalze im Reaktionsgemisch praktisch vollständig ungelöst, sondern lediglich suspendiert sind. Diese Salze müssen durch die Reaktionszone und durch die Kühlaggregate gefördert und nach der Reaktion mechanisch, z.B. mit Hilfe von Zentrifugen, abgetrennt werden. Dies bewirkt neben der bereits erwähnten Korrosion auch Erosionen, schlechten Wärmeübergang sowie Verstopfungen und Verkrustungen.

Um diese Nachteile eines Katalysatorkreislaufs zu vermeiden, hat man Vorschläge gemacht, die Katalysatorsalze im Reaktor suspendiert stationär zu halten und Methanol, CO und Sauerstoff in den Reaktor einzudosieren, während man aus dem Reaktor das gebildete Dialkylcarbonat und das Reaktionswasser zusammen mit im Überschuß eingesetztem Methanol abdestilliert (EP 0 413 215 A2). Dabei besteht das flüssige Reaktionsmedium im wesentlichen aus dem umzusetzenden Methanol (EP-0 413 215, Seite 3, Zeile 52), so daß das Molverhältnis zwischen Methanol und Cu-Salz sehr hoch ist (bevorzugt 1:0,01-0,05). Dies hat den Nachteil, daß die Reaktionsgeschwindigkeit relativ niedrig ist. Problematisch dabei ist auch die Notwendigkeit, eine niedrige Dimethylcarbonat-Konzentration einzustellen. Dies ist nicht leicht, da die Reaktion bei hohem Systemdruck durchgeführt wird und die Löslichkeiten von Dimethylcarbonat und auch von Wasser im Reaktionsmedium, das im wesentlichen aus Methanol besteht, sehr hoch sind. Dies bedeutet, daß die Abtrennungen von Dimethylcarbonat und Wasser mit einer relativ großen Inertgas- bzw. Methanolgasmenge erzwungen werden muß.

In einen neueren Verfahren gemäß DE-OS 41 38 755 wurde gezeigt, daß die Reaktionsgeschwindigkeit für die Oxycarbonylierung von Methanol wesentlich erhöht werden kann, wenn man Cu-Verbindungen in Form geschmolzener Salze bei 120° bis 300°C verwendet.

Der besondere Vorteil dieses Verfahrens kommt dann zur Geltung, wenn der Wassergehalt im Reaktionsgemisch niedrig gehalten werden kann (Patentanmeldung P 43 25 651.1 vom 30.7.1993).

Es wurde nun ein verbessertes Verfahren zur Herstellung von Dimethylcarbonat durch Umsetzen von Methanol mit Kohlenmonoxid und Sauerstoff in Gegenwart von Kupferverbindungen gefunden, das darin besteht, daß man die Umsetzung in Gegenwart geschmolzener Cu-Salze bei 120°C bis 300°C und 1 bis 70 bar in einer Reaktionsdestillationseinheit dergestalt durchführt, daß man das bei der Umsetzung

gebildete Reaktionswasser zumindest teilweise aus dem Abtriebsbereich oder der Sumpfphase dieser Reaktionsdestillationseinheit abtrennt.

Auf diese Weise kann das Reaktionswasser dem Reaktionssystem entzogen werden und die Cu-Salz-enthaltende Schmelze nach Abtrennung des Wassers und gegebenenfalls vorhandener Organika, wie Dimethylcarbonat, Methanol oder anderer im System vorhandener Spezies, in die Reaktionsdestillationseinheit zurückgeführt werden.

Auf diese Weise wird der Wassergehalt der Reaktionsmischung kontrolliert und auf niedrige Werte reduziert. Im allgemeinen arbeitet man bei einem Wassergehalt von unter 10 %. Es ist jedoch vorteilhaft, die Wasserkonzentration unter 6 Gew.-% in der Reaktionsmischung zu halten. Besonders günstig für hohen Umsatz bei gleichzeitig sehr hohen Selektivitäten ist es, die Reaktion unter 3 Gew.-% Werten zu fahren. In speziellen Durchführungsformen ist der Wassergehalt unter 1 %.

Bevorzugt wird das Verfahren der Erfindung bei Verwendung einer Cu-Salz enthaltenden Salzschmelze durchgeführt, wobei als Cu-Salz Cu-I- und Cu-II-Verbindungen sowie Gemische davon in Frage kommen. Grundsätzlich sind alle bekannten Cu-Salze, sofern sie in der Salzschmelze nur einigermaßen löslich sind, geeignet.

Geeignet sind neben den Halogeniden, wie z.B. den Chloriden oder den Bromiden, auch die Cyanide, Rhodanide, Sulfate, Nitrate, Carbonate, Acetate, Formiate, Oxalate, Alkoholate, z.B. Cu-methoxychlorid. Cu kann auch in Form komplexierter Verbindungen, wie den Acetylacetonaten, oder Cu-N-komplexen, wie Cu-Pyridin- oder auch Cu-Dipyridylkomplexen, eingesetzt werden.

Für die Salzschmelze nimmt man im allgemeinen Gemische von Salzen, die einen niedrigen Schmelzpunkt haben, d.h., die ein Eutektikum bilden. Es ist daher vorteilhaft, Mengenverhältnisse der Salze entsprechend der Zusammensetzung des Eutektiums zu verwenden. Solche Eutektika können von Cu-Salzen untereinander oder von Cu-Salzen und weiteren Salzen gebildet werden.

Neben den Cu-Salzen kann man daher im Prinzip alle chemisch inerten, oder auch im erfindungsgemäßen Sinne katalytisch wirksamen, d.h., die Aktivierungsenergie für die Oxycarbonylierung von Alkanolen herabsetzenden Salze verwenden. Neben Cu-Salzen kann man dabei eine breite Anzahl von Salzen oder salzartigen Verbindungen verwenden. In der Regel setzt man Gemische von Cu-Salzen und solchen salzartigen Verbindungen ein. Bevorzugt setzt man die Halogenide der 1. bis 3. Haupt- und Nebengruppen ein. Besonders geeignet sind die Chloride der Alkalimetalle, wie NaCl oder KCl, oder Chloride der Erdalkalien, wie $CaCl_2$ oder $MgCl_2$, sowie $ZnCl_2$. Aber auch die Verwendung von weniger häufigen Verbindungen, wie die Chloride von Thallium, Indium oder Gallium, ist möglich.

Eine gut geeignete Schmelze besteht beispielsweise aus Cu-I-Chlorid und KCl in unterschiedlichen Mengenverhältnissen. Im allgemeinen wählt man Mischungen mit einem hohen Gehalt an Cu-Verbindungen z.B. ein Gewichtsverhältnis von Cu-I-Chlorid zu KCl von 60 bis 75 zu 40 bis 25.

Die Reaktionstemperatur beträgt im allgemeinen etwa 120 °C bis 300 °C, bevorzugt 120 °C bis 180 °C, typische Reaktionstemperaturen liegen bei 140 °C bis 170 °C, vorteilhaft bei 145 °C bis 160 °C.

Die Reaktion kann bei Normaldruck durchgeführt werden. Um eine genügend hohe Reaktionsgeschwindigkeit zu erhalten, ist es jedoch zweckmäßig, bei höherem Druck, z.B. bei 5 bis 70 bar, bevorzugt bei 10 bis 50 bar, besonders bevorzugt bei 12 bis 22 bar, zu arbeiten.

Die Molverhältnisse der zum Einsatz gebrachten Reaktionskomponenten sind für die Reaktionsgeschwindigkeit und für die Selektivität der Reaktion von Bedeutung. Als Nebenprodukte treten bei Nichtbeachtung dieser Zusammenhänge beispielsweise das Dimethylacetal des Formaldehydes oder - was besonders störend ist - Methylchlorid auf.

Im allgemeinen wählt man einen molaren Überschuß von Methanol zu Kohlenmonoxid und wiederum einen Überschuß von Kohlenmonoxid zu Sauerstoff, höchstens jedoch molare Mengen von CO bzw. $O_2$. So wählt man Molverhältnisse von Alkanol zu CO und $O_2$ von 1:1-0,01:1-0,01, bevorzugt von 1:0,5-0,02:0,3-0,02. Dabei resultieren ein Methanolumsatz von beispielsweise 10 bis 50 % und ein CO-Umsatz von 10 bis 80 %. Sauerstoff wird im allgemeinen völlig umgesetzt. Bei der Mengendosierung müssen selbstverständlich die Explosionsgrenzen beachtet werden. Gegebenenfalls kann in Gegenwart von Inertgasen, wie $N_2$ oder $CO_2$, gearbeitet werden.

Der Sauerstoff kann beispielsweise in Form von atmosphärischer Luft oder von mit $O_2$ angereicherter Luft eingesetzt werden.

Die nicht umgesetzten Anteile von Methanol und CO können nach Abtrennung von Dimethylcarbonat und $H_2O$, gegebenenfalls auch von $CO_2$, zurückgeführt werden.

Das Verfahren der Erfindung kann diskontinuierlich durchgeführt werden. Es ist aber besonders für eine kontinuierliche, automatisierbare Produktion von Dimethylcarbonat geeignet. Als Reaktionsdestillationseinheit sind übliche Destillationskolonnen geeignet, bei denen die Cu-Salz-enthaltende Schmelze unterhalb der Kopfzone eindosiert wird und zusammen mit dem Rücklauf der Kolonne und gegebenenfalls eingesetztem

Methanol über die Einbauten oder die Packung der Kolonne nach unten fließt, wobei in den mittleren Bereich der Kolonne ein Gasgemisch aus Sauerstoff und CO, gegebenenfalls in Gegenwart von Inertgasen wie $N_2$ oder $CO_2$, eingeführt wird, das der Methanol enthaltenden Salzschmelze entgegenströmt.

Die Verweilzeit der Reaktionsgase in der Schmelze reicht im allgemeinen bei den üblichen Destillationseinheiten für einen ausreichenden Umsatz zu Dimethylcarbonat aus. Verweilzeiten von 0,5 bis 500 sec sind auf diese Art und Weise problemlos einstellbar. Gegebenenfalls kann man durch Vergrößerung der flüssigen Phase die Verweilzeit noch weiter erhöhen. Man kann neben den üblichen Packungen auch mit den üblichen Kolonnenböden arbeiten. Man kann aber auch z.B. durch Erhöhung der Ablaufwehre bei Bodenkolonnen die gewünschte Verweilzeit einstellen. Als Reaktionsdestillationseinheit können auch ein- bis mehrstufige Blasensäulen eingesetzt werden, die gegebenenfalls auch gerührt werden können.

Die unterhalb des Zutritts der Reaktionsgase CO und $O_2$ ablaufende Flüssigkeit enthält neben den Cu-Salzen Reaktionswasser und gegebenenfalls auch Dimethylcarbonat. Je nach den Bedingungen hinsichtlich Druck, Temperatur und Rücklaufverhältnissen kann die Menge an Dimethylcarbonat, die mit der Schmelze und dem Reaktionswasser dem Sumpf entgegenfließt, variieren bzw. festgelegt werden. Man kann also die Bedingungen in der Reaktionsdestillationseinheit so wählen, daß man das bei der Reaktion gebildete DMC weitgehend mit dem Methanol über Kopf enthimmt. Man kann die Bedingungen aber auch so wählen, daß man das DMC mit der Schmelze und dem Reaktionswasser aus dem Sumpf entnimmt. Die Sumpfphase wird nachfolgend, z.B. unter Entspannung, vom Reaktionswasser und gegebenenfalls vom DMC befreit. Die Schmelze fließt gegebenenfalls zusammen mit dem Frisch-Methanol in die Reaktionszone zurück. Das Reaktionswasser wird gegebenenfalls nach Abdestillieren von Organika, wie DMC, dem System entzogen.

In der Zone der Reaktionsdestillationseinheit oberhalb des Zulaufes der geschmolzenen Salze destillieren Methanol und gegebenenfalls Dimethylcarbonat sowie geringe Mengen flüchtiger Nebenprodukte, wie z.B. Formaldehyddimethylacetal.

Das Kopfprodukt wird nach Kondensation gegebenenfalls teilweise in die Reaktionsdestillationseinheit zurückgeleitet. Der nicht zurückgeleitete Anteil des Kondensats wird der Kolonne entnommen. Aus diesem der Kolonne entnommenen Anteil wird in an sich bekannter Weise reines DMC isoliert und das Methanol in die Reaktionszone zurückgeführt.

Die in der Reaktionszone entstehende Reaktionswärme wird durch verdampfende Reaktionspartner abgeführt.

Bei der Aufarbeitung des Reaktionsgemisches gegebenenfalls auftretende Mischungen von Methanol und Dimethylcarbonat können als Gemische in die Reaktionszone zurückgeführt werden und dienen als Methanolquelle.

Auf diese Art und Weise kann das in die Reaktionszone eingeführte Methanol bereits größere Mengen an Dimethylcarbonat enthalten, die beispielsweise der Zusammensetzung des Azeotrops Methanol/Dimethylcarbonat entsprechen, aber auch höhere Mengen an DMC enthalten können.

Der besondere Vorteil des erfindungsgemäßen Verfahrens liegt darin, daß man Reaktionsgemische mit hoben Dimethylcarbonatgehalten, beispielsweise 10 bis 90 Gew.-%, vorzugsweise 50 bis 70 Gew.-% erhält, die die Isolierung des reinen Dimethylcarbonates in besonders einfacher und wirtschaftlicher Weise gestatten.

Als Werkstoffe für die Reaktionsdestillationseinheit eignen sich beispielsweise korrosionsbeständige Edelstähle, Stahl-Emaille, Glas, Graphit oder Sondermetalle, wie Tantal.

Das erfindungsgemäße Verfahren kann auch technisch im größeren Maßstab durchgeführt werden. Dabei kann man beispielsweise gemäß Fig. 1 in einer Reaktionsdestillationseinheit (A) Methanol (1) zusammen mit der Salzschmelze (7), dem Azeotrop DMC/Methanol (11) einem Gasstrom aus CO (2) und $O_2$ (3) zusammen mit dem Kreisgas (4) entgegenleiten.

Aus dem Kolonnensumpf der Einheit (A) wird nach Entspannung das Reaktionswasser (9) abdestilliert. Die von Wasser befreite Schmelze wird in die Kolonne (A) zurückgeführt.

Am Kopf der Kolonne (A) wird nach Kondensation ein Teilstrom der kondensierten Phase als Rücklauf auf die Kolonne gegeben. Der andere Teilstrom, der ca. 60 % DMC (Rest Methanol) enthält,fließt in die Azeotropkolonne (B), bei der bei 10 bar Dimethylformal (10) über Kopf geht. Der Sumpfaustrag der Azeotropkolonne (B) geht in die Dimethylcarbonat-Reinkolonne (C), aus der reines Dimethylcarbonat (8) als Seitenstrom in der Nähe des Sumpfes gewonnen wird. Das Kopfprodukt der DMC-Reinkolonne (C) besteht im wesentlichen aus dem Azeotrop aus Dimethylcarbonat und Methanol und wird zusammen mit der Salzschmelze in die Reaktionszone zurückgeführt.

Die nichtkondensierbaren Anteile (4) der Reaktionsdestillationseinheit (A) werden mittels eines Kompressors in die Reaktionszone zurückgeführt. Ein Teilstrom des Kreisgases (5) wird zur Ausschleusung von Inertgasen abgenommen.

Man kann aber auch gemäß Fig. 2 so verfahren, daß man in die Reaktionsdestillationseinheit (A) Methanol (1) zusammen mit der Schmelze (7) leitet. Diesem durch die Einbauten der Kolonne nach unten fließenden Strom strömt das Gemisch der Reaktionsgase $O_2$ (3) und CO (2) zusammen mit dem Kreisgas (4) entgegen. Unterhalb der Reaktionszone fließt ein Gemisch aus geschmolzenen Salzen, Wasser und Dimethylcarbonat in den Sumpf der Reaktionsdestillationskolonne, aus dem dieser Produktstrom, bestehend aus Salzschmelze, Wasser und Dimethylcarbonat, entnommen wird. Aus diesem Sumpfabzug werden Wasser und Dimethylcarbonat abdestilliert und in der Wasserabtrennungskolonne (B) fraktioniert, wobei im Sumpf Wasser (9) erhalten wird.

Über Kopf destilliert das Azeotrop Wasser/Dimethylcarbonat. Nach Phasentrennung fließt das abgeschiedene Wasser in die Kolonne zurück. Die organische obere Phase enthält im wesentlichen Dimethylcarbonat, das in der DMC-Reinkolonne (C) als Seitenstrom in der Nähe des Sumpfes (8) entnommen wird.

Oberhalb der Reaktionszone der Reaktionsdestillationseinheit (A) destillieren im wesentlichen Methanol und flüchtige Nebenprodukte, wie Formaldehyddimethylacetal. Das Kondensat wird im wesentlichen als Produktstrom (6) als Rücklauf auf die Reaktionsdestillationskolonne (A) gegeben. Aus einem Teilstrom des Kondensates wird zur Ausschleusung flüchtiger Nebenprodukte ein kleinerer Teilstrom (purge, 10) entnommen. Die nicht kondensierbaren Anteile des Kopfproduktes (4) werden mit Hilfe eines Kompressors in die Reaktionszone zurückgeführt. Aus diesem Kreisgas wird zur Ausschleusung von Inertgasen ein Teilstrom (5) entnommen.

## Mengenbilanz für das DMC-Salzschmelze-Verfahren

Beispiel nach Fig. 1

| | Methanol Zulauf | Kohlenmonoxid Zulauf | Sauerstoff Zulauf | Kreisgas | Purge | Rücklauf | Produkt | Reaktionswasser | Formaldehyd-dimethylazetal (FDA) | Azeotrop-Rücklauf |
|---|---|---|---|---|---|---|---|---|---|---|
| | 1 | 2 | 3 | 4 | 5 | 6 | 8 | 9 | 10 | 11 |
| Methanol | 2931 100,0% | 0,0% | 0,0% | 0,0% | 0,0% | 18016 40,0% | 0,0% | 0,0% | 0,0% | 3023 85,0% |
| CO | 0,0% | 1262 100,0% | 0,0% | 3578 40,6% | 23 40,6% | 0,0% | 0,0% | 0,0% | 0,0% | 0 0,0% |
| N2 | 0,0% | 0,0% | 0,0% | 0,0% | 0,0% | 0,0% | 0,0% | 0,0% | 0,0% | 0 0,0% |
| O2 | 0,0% | 0,0% | 735 100,0% | 773 8,8% | 5 8,8% | 0,0% | 0,0% | 0,0% | 0,0% | 0 0,0% |
| DMC | 0,0% | 0,0% | 0,0% | 0,0% | 0,0% | 27024 60,0% | 4000 100,0% | 0,0% | 0,0% | 533 15,0% |
| Formal | 0,0% | 0,0% | 0,0% | 0,0% | 0,0% | 0,0% | 0,0% | 0,0% | 68 100,0% | 0 0,0% |
| CO2 | 0,0% | 0,0% | 0,0% | 4464 50,6% | 29 50,6% | 0,0% | 0,0% | 0,0% | 0,0% | 0 0,0% |
| CuCl/KCl | 0,0% | 0,0% | 0,0% | 0,0% | 0,0% | 0,0% | 0,0% | 0,0% | 0,0% | 0 0,0% |
| Wasser | 0,0% | 0,0% | 0,0% | 0,0% | 0,0% | 0,0% | 0,0% | 832 100,0% | 0,0% | 0 0,0% |
| | 2931 100,0% | 1262 100,0% | 735 100,0% | 8814 100,0% | 58 100,0% | 45040 100,0% | 4000 100,0% | 832 100,0% | 68 100,0% | 3556 100,0% |

Beispiel nach Fig. 2

| | Methanol Zulauf | Kohlenmonoxid Zulauf | Sauerstoff Zulauf | Kreisgas | Purge | Rücklauf | Produkt | Reaktionswasser | Formaldehyd-dimethylazetal (FDA) |
|---|---|---|---|---|---|---|---|---|---|
| | 1 | 2 | 3 | 4 | 5 | 6 | 8 | 9 | 10 |
| Methanol | 2931 100,0% | 0,0% | 0,0% | 0,0% | 0,0% | 38284 85,0% | 0,0% | 0,0% | 0,0% |
| CO | 0,0% | 1262 100,0% | 0,0% | 3578 40,6% | 23 40,6% | 0,0% | 0,0% | 0,0% | 0,0% |
| N2 | 0,0% | 0,0% | 0,0% | 0,0% | 0,0% | 0,0% | 0,0% | 0,0% | 0,0% |
| O2 | 0,0% | 0,0% | 735 100,0% | 773 8,8% | 5 8,8% | 0,0% | 0,0% | 0,0% | 0,0% |
| DMC | 0,0% | 0,0% | 0,0% | 0,0% | 0,0% | 6756 15,0% | 4000 100,0% | 0,0% | 0,0% |
| Formal | 0,0% | 0,0% | 0,0% | 0,0% | 0,0% | 0,0% | 0,0% | 0,0% | 68 100,0% |
| CO2 | 0,0% | 0,0% | 0,0% | 4464 50,6% | 29 50,6% | 0,0% | 0,0% | 0,0% | 0,0% |
| CuCl/KCl | 0,0% | 0,0% | 0,0% | 0,0% | 0,0% | 0,0% | 0,0% | 0,0% | 0,0% |
| Wasser | 0,0% | 0,0% | 0,0% | 0,0% | 0,0% | 0,0% | 0,0% | 832 100,0% | 0,0% |
| | 2931 100,0% | 1262 100,0% | 735 100,0% | 8814 100,0% | 58 100,0% | 45040 100,0% | 4000 100,0% | 832 100,0% | 68 100,0% |

## Patentansprüche

1. Verfahren zur Herstellung von Dimethylcarbonat aus Methanol, Kohlenmonoxid und Sauerstoff in Gegenwart von Kupferverbindungen, dadurch gekennzeichnet, daß man die Umsetzung in Gegenwart

geschmolzener Cu-Salze bei 120 °C bis 300 °C und 1 bis 70 bar in einer Reaktionsdestillationseinheit dergestalt durchführt, daß man das bei der Reaktion gebildete Wasser zumindest teilweise aus dem Abtriebsbereich oder der Sumpfphase dieser Reaktionsdestillationseinheit abtrennt.

**2.** Verfahren nach Anspruch 1, dadurch gekennzeichnet, daß der Wassergehalt in der Reaktionszone auf einen Wert von unter 6 Gew.-%, bevorzugt von unter 3 Gew.-%, ganz besonders unter 1 Gew.-% gehalten wird.

**3.** Verfahren nach Anspruch 1, dadurch gekennzeichnet, daß eine Schmelze von Cu-I-chlorid und KCl eingesetzt wird.

**4.** Verfahren nach Anspruch 3, dadurch gekennzeichnet, daß ein Gewichtsverhältnis CuCl : KCl = 60 bis 75 : 40 bis 25 eingestellt wird.

**5.** Verfahren nach Anspruch 1, dadurch gekennzeichnet, daß ein Molverhältnis von Alkanol:CO:$O_2$ = 1:1 - 0,01:1 - 0,01, bevorzugt 1:0,5 - 0,02:0,3 - 0,02 eingestellt wird.

**6.** Verfahren nach Anspruch 1-5, dadurch gekennzeichnet, daß man das Reaktionswasser gemeinsam mit gebildetem Dimethylcarbonat aus dem Sumpfgemisch der Reaktionsdestillationseinheit herausdestilliert und das dabei erhaltene Destillat in Wasser und Dimethylcarbonat trennt.

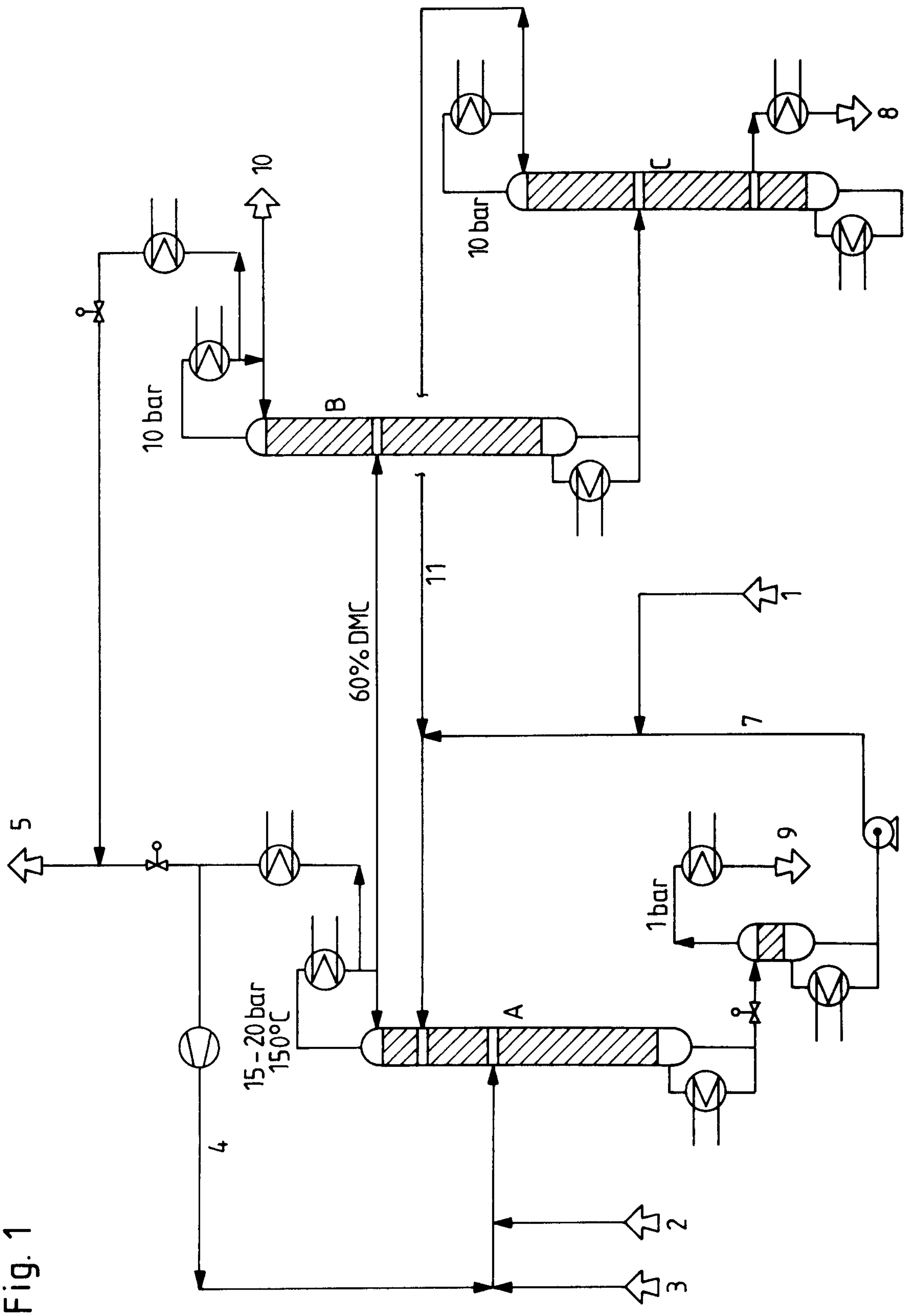

Fig. 1

Fig. 2

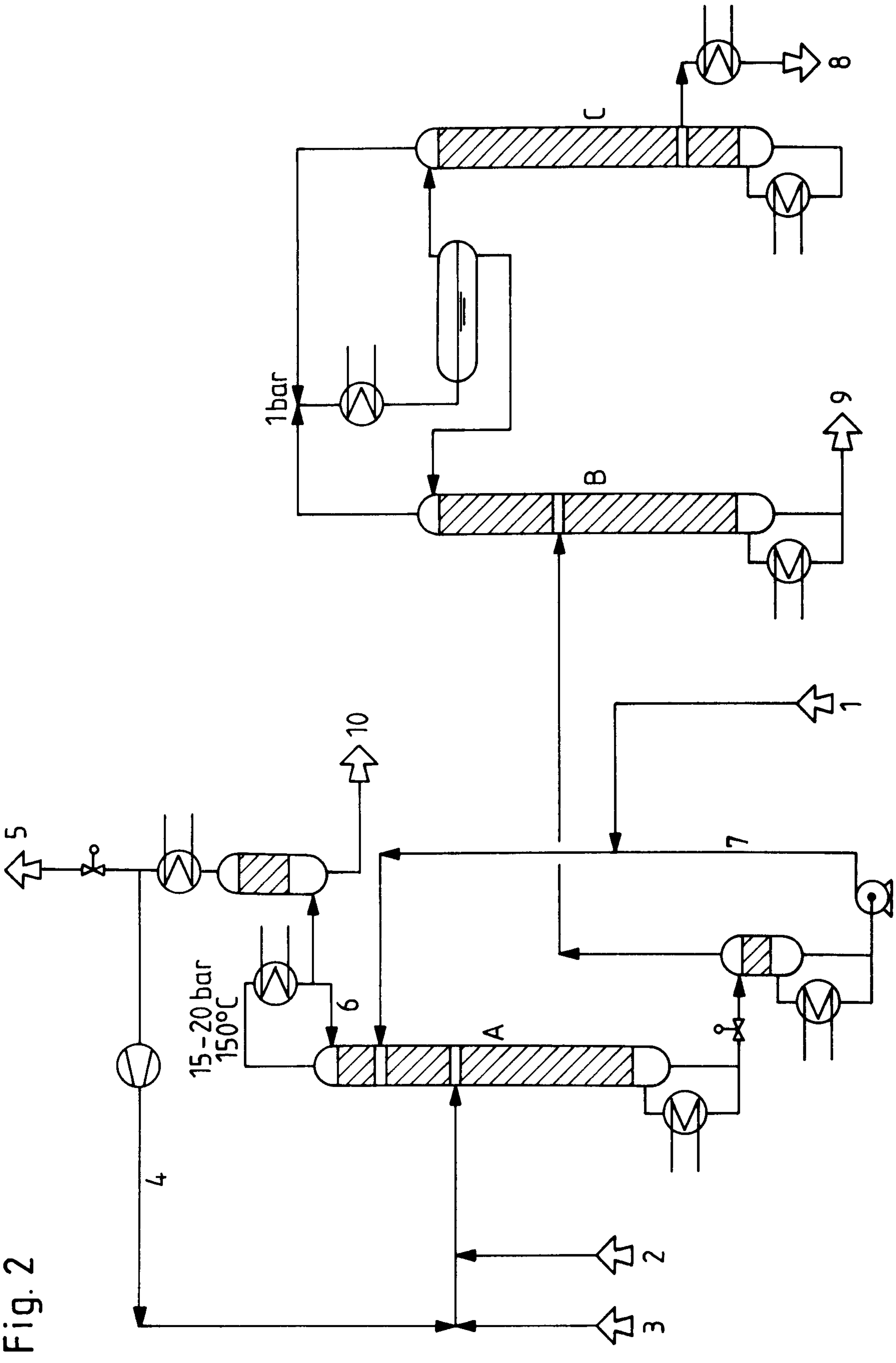

Europäisches Patentamt

# EUROPÄISCHER RECHERCHENBERICHT

Nummer der Anmeldung
EP 94 11 9607

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| E<br><br>D,E | EP-A-0 636 601 (BAYER AG)<br>* Ansprüche 1-3,7 *<br>& DE-A-43 25 651 | 1-3 | C07C68/00<br>C07C69/96 |
| A<br><br>D | EP-A-0 544 162 (BAYER AG)<br>* Ansprüche 1,6 *<br>& DE-A-41 38 755 | 1,3 | |
| A | EP-A-0 134 668 (IMPERIAL CHEMICAL INDUSTRIES PLC)<br>* Anspruch 1 * | 1 | |
| | | | **RECHERCHIERTE SACHGEBIETE (Int.Cl.6)** |
| | | | C07C |

Der vorliegende Recherchenbericht wurde für alle Patentansprüche erstellt

| Recherchenort | Abschlußdatum der Recherche | Prüfer |
|---|---|---|
| BERLIN | 14. März 1995 | Kapteyn, H |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)